# EUROPÄISCHE PATENTSCHRIFT

(11) **EP 1 385 536 B1**
(45) Veröffentlichungstag und Bekanntmachung des Hinweises auf die Patenterteilung: **05.10.2005**
(21) Anmeldenummer: 02766574.4
(22) Anmeldetag: 30.04.2002
(51) Int. Cl.: A61K 38/38, A61K 31/355, A61P 25/16, A61P 25/28, A61P 25/14, A61P 9/10

(54) **PRÄPARATIONEN VON VITAMIN E IN KOMBINATION MIT AFAMIN**
PREPARATIONS CONTAINING A COMBINATION OF VITAMIN E AND AFAMIN
PREPARATIONS DE VITAMINE E EN COMBINAISON AVEC L'AFAMINE

(30) Priorität: 30.04.2001 AT 6962001
(43) Veröffentlichungstag der Anmeldung: 04.02.2004
(73) Patentinhaber: Vitateq Biotechnology GmbH, 6020 Innsbruck (AT)
(72) Erfinder: BECKER-ANDRE, Michael, A-6020 Innsbruck (AT); DIEPLINGER, Hans, A-6020 Innsbruck (AT); TEUNISSEN, Charlotte, 3818 DM Amersfoort (NL); JERKOVIC, Lidija, 8055 Graz (AT)
(74) Vertreter: Sonn & Partner Patentanwälte
(86) Internationale Anmeldenummer: PCT/AT2002/000132
(87) Internationale Veröffentlichungsnummer: WO 2002/087604

(56) Entgegenhaltungen:
- WO-A-01/01148
- WO-A-95/27059
- JERKOVIC L ET AL: "AFAMIN AND VITAMIN E IN FOLLICULAR FLUID OF PATIENTS UNDERGOING IVF" HUMAN REPRODUCTION, IRL PRESS, OXFORD, GB, Bd. 14, Nr. ABSTR BOOK 1, 26. Juni 1999 (1999-06-26), Seiten 203-204, XP001056307 ISSN: 0268-1161
- TEUNISSEN C E ET AL: "Cognitive performance in combination with serum markers in an aging population: a 6 year follow-up study as part of the Maastricht Aging Study." SOCIETY FOR NEUROSCIENCE ABSTRACTS, Bd. 26, Nr. 1-2, 2000, Seiten Abstract No.-575.3, XP001087735 30th Annual Meeting of the Society of Neuroscience;New Orleans, LA, USA; November 04-09, 2000 ISSN: 0190-5295

## Beschreibung

Die Erfindung betrifft Präparationen von Vitamin E in Kombination mit Afamin.

Afamin ist ein 87 kDa-Protein, welches zur Albumin-Gruppe zählt und viele strukturelle und biochemische Gemeinsamkeiten mit den Proteinen dieser Gruppe, d.h. mit humanem Serumalbumin, humanem α-Fetoprotein oder humanem Vitamin-D-Bindungsprotein aufweist. Afamin wurde bereits kloniert und sequenziert und steht daher auch in rekombinanter Form zur Verfügung (WO 95/270959).

Über die Funktion von Afamin ist - abgesehen von seinen Sequenzhomologien - wenig bekannt. Es wurde die Möglichkeit diskutiert, dass Afamin Sterolbindungsstellen aufweist, jedoch Aktin wahrscheinlich nicht bindet. Aufgrund der gegebenen, aber nicht überragenden Ähnlichkeit zwischen Afamin und Albumin wird es als zweifelhaft angesehen, dass diese Proteine dieselben Liganden binden (Lichenstein et al., The Journal of Biological Chemistry, 269 (27) (1994), S.18149-18154).

Biochemische und physiologische. Untersuchungen ergaben, dass Afamin Vitamin-E-bindende Eigenschaften aufweist und nicht nur im Blut, sondern auch in anderen Körper- oder Organflüssigkeiten, wie beispielsweise Cerebrospinal-, Follikel- und Samenflüssigkeit, auftritt. Die Verwendung von Afamin zur Fertilitätsbestimmung von Säugetieren ist in der WO 01/01148 A1 beschrieben.

Vitamin E bzw. die Vitamin-E-Gruppe wird als Sammelbezeichnung für fettlösliche, natürlich vorkommende Verbindungen mit einem Chroman-Grundgerüst und einer C₁₆-Seitenkette (Tocopherole) verwendet. Tocopherole sind Chroman-6-ole (3,4-Dihydro-2H-1-benzo-pyran-6-ole), die in 2-Stellung mit einem 4,8,12-Trimethyltridecylrest substituiert sind. Tocopherole sind schwach gelblichrötlich ölige Flüssigkeiten, die unlöslich in Wasser, jedoch löslich in Fetten und Ölen sowie den üblichen Lösungsmitteln für Fette sind. Man unterscheidet u.a. α-, β-, γ-, δ- und ε-Tocopherole, wobei letzteres noch über die ursprüngliche ungesättigte Prenylseitenkette verfügt, sowie α-Tocochinone und Hydrochinone, bei denen das Pyranringsystem geöffnet ist. Das häufigste und am stärksten wirksame natürliche Tocopherol ist das α-Tocopherol (2R,4'R,8'R)-Form (Trivialname: RRR-α-Tocopherol), das wie alle anderen Stereoisomeren synthetisch zugänglich ist (Römpp Chemie-Lexikon, 10. Aufl., S. 4572/4573 und 4878-4886).

Ursprünglich als Antisterilitäts-Vitamin beschrieben, überwiegt heute die Ansicht, dass die Vitamine der E-Gruppe hauptsächlich als Fänger von Hyperoxid- und Peroxidalradikalen als Antioxidans für ungesättigte Fettsäuren für LDL, für Vitamin A und Karotine fungiert. Auch eine wichtige Rolle bei der Entzündungshemmung und bei der Immunfunktion wird Vitamin E zugeschrieben.

Vitamin-E-Präparate werden daher hauptsächlich in durchblutungsfördernden und lipidsenkenden Mitteln für Mensch und Tier eingesetzt.

Die Aufgabe der vorliegenden Erfindung besteht darin, neue medizinische Verwendungen für Afamin zur Verfügung zu stellen. Weiters sollen Vitamin-E-Präparate mit verbesserter Wirkung, insbesondere hinsichtlich ihrer antioxidativen Eigenschaften, zur Verfügung gestellt werden.

Diese Aufgabe wird erfindungsgemäß gelöst durch ein Präparat, enthaltend Afamin, insbesondere in Kombination mit Vitamin E, welches zur Herstellung einer Präparation zur Behandlung von oxidativem Stress, insbesondere zur Behandlung von neurodegenerativen Erkrankungen, verwendet werden kann. Es hat sich erfindungsgemäß überraschend gezeigt, dass durch Kombination von Vitamin E und Afamin eine Vitamin-E-Präparation mit verbesserten Eigenschaften zur Verfügung gestellt werden kann, insbesondere eine Vitamin-E-Präparation mit erhöhter antioxidativer Wirkung.

Obgleich für Afamin eine ganze Reihe verschiedenster Funktionen postuliert worden ist (v.a. als Transportsystem für Fettsäuren, Hormone, Enzyme, Farbstoffe, Spurenmetalle und Medikamente sowie als direktes Antioxidans (vgl. WO 95/27059)), hat sich zusätzlich erfindungsgemäß gezeigt, dass Afamin selbst (sogar ohne Vitamin E) eine neuroprotektive Funktion hat, die durch eine überraschende synergistische Wirkung mit Vitamin E gesteigert wird. Demgemäß kann vor allem das erfindungsgemäß zur Verfügung gestellte Kombinationspräparat von Afamin und Vitamin E zur Behandlung von oxidativem Stress für eine breite Anwendungspalette zur Verfügung gestellt werden. Es hat sich erfindungsgemäß gezeigt, dass sich die Kombination von Afamin und Vitamin E besonders zur Behandlung von neurodegenerativen Erkrankungen, die mit oxidativem Stress verbunden sind, eignet.

Dies war insofern überraschend, als die bislang für Afamin postulierten Verwendungen (s. WO 95/27059, worin u.a. die Verwendung bei rheumatoider Arthritis, ARDS, Sepsis, Ateriosklerose usw., für möglich erachtet worden sind, ohne jedoch hiefür Belege oder plausible Modellversuche vorlegen zu können) bislang nicht bestätigt wurden. Umso unerwarteter war es, dass Afamin, vor allem in Kombination mit Vitamin E (und eben nicht in Kombination mit beispielsweise Vitamin D, einem bekannten Bindungspartner eines anderen Proteins aus der Albumin-Gruppe), eine - gegenüber den Einzelkomponenten - signifikant verbesserte antioxidative Wirkung aufweist.

Unter Vitamin E werden erfindungsgemäß sämtliche natürliche und synthetische biologisch wirksame Tocopherol-Präparate, wie eingangs geschildert, angesehen, insbesondere aus natürlichen Quellen stammende Tocopherol-Präparate, insbesondere aus Pflanzenölen, wie z.B. Soja, Weizen, Mais, Reis, Baumwolle, Luzerne und Nüsse, sowie aus Früchten und Gemüse, z.B. Himbeeren, Bohnen, Erbsen, Fenchel, Paprika, Schwarzwurzen und Sellerie. Auch die synthetisch hergestellten Tocopherol-Präparate, einschließlich Derivate derselben, wie z.B. Tocopherylacetat, -succinat-, -nicotinat und -poly(oxyethylen)succinat sind im Rahmen der vorliegenden Erfindung einsetzbar (vgl. Römpp Chemie-Lexikon, Seiten 4572/4573).

Unter Afamin werden erfindungsgemäß alle Polypeptide, die eine biologische Aktivität mit dem natürlichen Afamin gemein haben, angesehen, insbesondere verschiedene glykosylierte, deglykosylierte, unglykosylierte, lipidierte oder entlipidierte Formen sowie mit rekombinanten Technologien hergestellte Varianten von Afamin. Eine ausführliche strukturelle Definition von Afamin ist beispielsweise in der WO 95/27059 angeführt, die hierin durch Bezugnahme ausdrücklich mit aufgenommen ist. Zusätzlich werden rekombinant oder proteolytisch erzeugte isolierte Subdomänen sowie chemisch derivatisierte oder modifzierte Formen (beispielsweise durch Azetylierung, z.B. an Lysin-Seitenketten) und rekombinant oder chemisch erzeugte erweiterte Versionen des Proteins beispielsweise durch Anhängen von funktionalen Peptiden, die die Blut-Hirnschranken-Gängigkeit erhöhen (wie z.B. in Mazel et al (Anticancer Drugs 12(2) (2001), 107-116), Temsamani et al. (Pharm. Sci. Technol. Today 3(5) (2000), 155-162), Rousselle et al. (Mol. Pharmacol. 57(4) (2000), 679-686), Rousselle et al. (J. Pharmacol. Exp. Ther. 296(1) (2001), 124-131) beschrieben) mit einbezogen.

Freie Radikale sind in einer Reihe von akuten und chronischen neurologischen Störungen, insbesondere bei fokaler Ischämie, Trauma, Epilepsie, Huntingtonscher Erkrankung, Alzheimerscher Erkrankung, amyotropischer Lateralsklerose (ALS), AIDS, Demenz und anderen neurodegenerativen Erkrankungen involviert. Weiters existiert eine zunehmende Anzahl an Untersuchungen, die eine Beteiligung von reaktiven Sauerstoff-Spezies (reactive oxygen species, ROS) in traumatischen Gehirnverletzungen nahelegt. Auch die Empfindlichkeitsbeeinträchtigung von verschiedenen Rezeptorsystemen mit zunehmendem Alter weist auf eine zunehmende Verschlechterung der Reaktionen auf oxidativen Stress hin.

Glutamat-induzierte Zytotoxizität ist ein nützliches Modellsystem, um Verbindungen hinsichtlich ihrer antioxidativen Aktivität zu testen (Kabayashi et al., Free Radic Res., 32 (2000),115-124). Glutamat-induzierte Zytotoxizität in HT-4 neuronalen Zellen wurde auf oxidativen Stress, der durch Verminderung von zellulärem Glutathion verursacht wird, zurückgeführt. Glutamat induziert Apoptose in cortikalen Rattenneuronen und in der Hippocampus-HT-22-Zelllinie aus Mäusen, indem die Cysteinaufnahme blockiert wird, wodurch intrazelluläres Glutathion vermindert wird. Dies resultiert wiederum in der Anreicherung von ROS. Eine geringe Konzentration von α-Tocopherolen hat sich als sehr wirksam zum Schutz der neuronalen Zellen gegen Zytotoxizität erwiesen.

Neuropathologien der Huntingtonschen Erkrankung entstehen aus einer exzessiven Aktivierung von Glutamat-gekuppelten Ionenkanälen, wodurch Neuronen durch oxidativen Stress abgetötet werden.

α-Tocopherol könnte oxiradikale Schädigungen der Zellmembran verhindern und eine Verlangsamung des Ablaufs der Huntingtonschen Erkrankung wurde für derartige Präparate diskutiert. Insbesondere könnte eine Antioxidans-Therapie die Geschwindigkeit der Abnahme der motorischen Fähigkeiten im Zuge der Huntingtonschen Erkrankung verlangsamen (Peyser et al., Am.J.Psychiatry, 152 (1995), 1771-1775).

Die Expression von zwei mutierten Superoxid-Dismutasen (SODs), die in Zusammenhang mit familiärer ALS stehen, verursachen das Absterben von differenzierten PC12-Zellen, oberen cervikalen Ganglienneuronen und pyramidalen Neuronen im Hippocampus (Chadge et al., J.Neurosci, 17 (22) (1997), 8756-8766). Beim Zelltod können viele Merkmale, die typisch für Apoptose sind, festgestellt werden. Dieser Zelltod könnte durch effiziente Behandlung mit Vitamin E verhindert werden (Chadge et al., J.Neurosci, 17 (22) (1997), 8756-8766).

Das Striatum enthält eine hohe Konzentration von oxidierbarem Dopamin. Ein älterer Organismus zeigt eine verminderte Fähigkeit, gegenüber oxidativem Stress zu antworten, wodurch dieser Bereich sehr anfällig wird für Schäden, die durch freie Radikale bedingt sind. Dopamin-Neuronen sind speziell anfällig für derartige Schäden und Erkrankungen. Ein Verlust von Dopamin-Neuronen ist beispielsweise mit der Parkinsonschen Erkrankung verbunden, wobei hierbei schädliche sauerstofffreie Radikale akkumuliert werden. α-Tocopherole könnten Zellen gegen zytotoxische Effekte, die durch Dopamin und L-Dopa verursacht werden, schützen (Ebadi et al, Prog.Neurobiol. 48(1) (1996), 1-19).

Es existieren auch überzeugende epidemiologische und in vitro - Evidenz, dass in Personen mit Down-Syndrom chronischer oxidativer Stress auftritt (Javanovic et al., Free Radic Res., 9 (1998), 1044-1048). Derartige Patienten entwickeln eine Alzheimer-ähnliche Veränderung im Gehirn ab einem Alter von 30 bis 40 Jahren. In in vitro-Studien konnte gezeigt werden, dass die verminderte Lebensfähigkeit von Down-Syndrom-Neuronen durch Antioxidantien, wie Vitamin E, verändert werden kann. Die Aufnahme derartiger Oxidantien mit der Nahrung allein scheint aber keine ausreichende Veränderung bzw. Verbesserung im Hinblick auf die Biomarker für oxidativen Stress zu bewirken.

Oxidative Schäden wurden auch in Zusammenhang mit akuten degenerativen Erkrankungen, wie Epilepsie, Trauma und zerebralen Ischämie-Zuständen, wie z.B. bei Schlaganfällen, in Zusammenhang gebracht. Bei diesen neurologischen Erkrankungen wurde die Antioxidantien-Therapie immer als günstig angesehen, jedoch fehlten bislang effiziente Mittel hiefür.

Haloperidol, ein Dopaminrezeptor-Antagonist, wird häufig zur Behandlung von Schizophrenie und anderen affektiven Störungen verschrieben. Oxidativer Stress gilt als eine der hauptsächlichen klinischen Nebenwirkungen von Haloperidol. Diese Verbindung ist für HT22-Zellen aus Mäuse-Hippocampus in einer Konzentrationsabhängigen weise tödlich und verursacht den Zelltod durch oxidativen Stress. HP-induzierter oxidativer Zelltod kann am besten durch Vitamin E-Präparate verhindert werden (Post et al., J. Neurosci, 18(20) (1998), 8236-8246); Sagara, J. Neurochem, 71(3) (1998), 1002-1012). Auf molekularer Ebene induziert Haloperidol spezifisch die Aktivität des Redox-sensitiven Transkriptionsfaktor NF-κB. Diese verstärkte NF-κB-Aktivität konnte durch neuroprotektive Antioxidantien blockiert werden.

Obgleich die Wirkung von antioxidativen Enzymen bei der Alzheimerschen Erkrankung widersprüchlich sind, wurden von vielen Forschern Veränderungen in Glutathion-Peroxidase- oder in Superoxid-Dismutase-Aktivitäten beobachtet und viele Anhaltspunkte sprechen dafür, dass oxidativer Stress auch in Alzheimer-Patienten eine große Rolle spielt. Vor allem konnte gezeigt werden, dass Substanzen, die in der Lage sind, freie Radikale einzufangen, wie Vitamin E, Selegilin und Gynko bilobaextract Egb 761, positive Wirkung bei der therapeutischen Behandlung von Alzheimer-Patienten zeigen (Christen, Am.J.Clin.Nutr., 71(2) (2000), 621s-629s).

Aus diesen Gründen wird das erfindungsgemäße Afamin, insbesondere aber das Kombinationspräparat aus Afamin und Vitamin E bevorzugterweise zur Behandlung der Alzheimerschen Erkrankung, der Huntington-Erkrankung oder der amyotropischen Lateralsklerose eingesetzt.

Das erfindungsgemäße Kombinationspräparat ist daher besonders geeignet zur Behandlung von neurodegenerativen Demenzen, wie sie beispielsweise bei diesen Erkrankungen auftreten.

Gemäß einer weiteren Ausführungsform betrifft die vorliegende Erfindung die Verwendung von Afamin, insbesondere in Kombination mit Vitamin E, zur Herstellung einer Präparation zur Behandlung akuter neurodegenerativer Erkrankungen, insbesondere zur Behandlung von Epilepsie, Trauma und zerebraler Ischämie (z.B. einem Schlaganfall).

Aus den oben dargelegten Gründen eignet sich Afamin bzw. das erfindungsgemäße Kombinationspräparat auch zur Behandlung der Parkinsonschen Erkrankung oder zur Behandlung der der Alzheimerschen Erkrankung ähnlichen Veränderungen bei Patienten mit Down-Syndrom.

Gemäß einem weiteren Aspekt der vorliegenden Erfindung kann mit Afamin bzw. dem erfindungsgemäßen Kombinationspräparat auch oxidativer Stress, der als Nebenwirkung einer Medikamentenbehandlung auftritt, verhindert werden. Hierbei kann das Afamin bzw. das erfindungsgemäße Kombinationspräparat zusammen mit dem jeweiligen Medikament vorgesehen werden oder aber getrennt davon verabreicht werden.

Für all diese Indikationen ist in der Fachwelt bereits eine positive therapeutische oder prophylaktische Wirkung beschrieben oder zumindest plausibel postuliert worden. Mit der erfindungsgemäßen Kombinationspräparation, mit welcher die antioxidative Wirkung von Vitamin-E-Präparaten entscheidend verbessert werden, können diese Indikationen erfindungsgemäß effizienter behandelt, verhindert oder zumindest in deren Progression verzögert werden.

Afamin bzw. das erfindungsgemäße Kombinationspräparat können für all diese Erkrankungen sowohl therapeutisch als aus prophylaktisch eingesetzt werden, wodurch sich der Ausbruch der klassisch festgestellten Symptome hintanhalten lässt bzw. der Krankheitsverlauf verzögert wird. Demgemäß betrifft die vorliegende Erfindung auch die Verwendung von Afamin bzw. des erfindungsgemäßen Kombinationspräparats zur Herstellung einer neuroprotektiven Präparation.

Ein weiterer Aspekt der vorliegenden Erfindung betrifft die Verwendung von Afamin, insbesondere in Kombination mit Vitamin E, zur Herstellung einer Präparation zur Behandlung von Infertilitätsstörungen. Es hat sich erfindungsgemäß gezeigt, dass mit Hilfe von Afamin Infertilitätsstörungen wirksam behandelt werden können. Auch kann das ursprünglich als Fertilitätsfaktor bekannt gewordene Vitamin E durch Kombination mit Afamin in seiner an sich bekannten Fertilitätswirkung entscheidend verbessert werden.

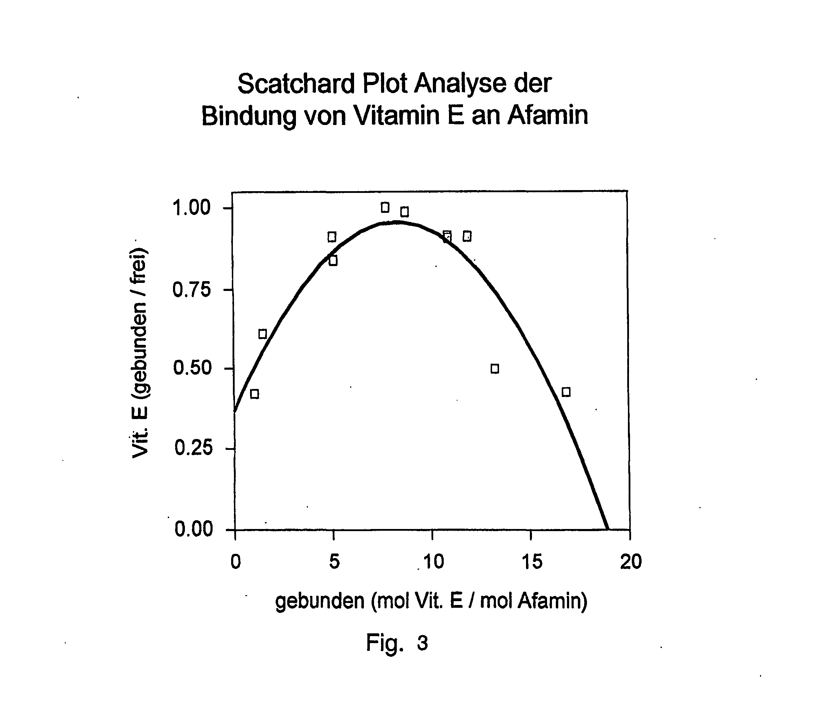

Gemäß einem weiteren Aspekt betrifft die vorliegende Erfindung eine Vitamin-E-Präparation mit erhöhter antioxidativer Wirkung, welche sich dadurch auszeichnet, dass sie neben einem aus synthetischen oder natürlichen Quellen hergestellten Vitamin-E-Präparat zusätzlich Afamin enthält. Bevorzugterweise weisen dabei Afamin und Vitamin E ein molares Verhältnis von zwischen 20 bis 100 Vitamin E / Afamin auf. Dieses Verhältnis berücksichtigt die theoretische maximale Beladung eines Afaminmoleküls durch 18 Moleküle Vitamin E (s. Fig. 3). Insbesondere kann dabei Afamin eingesetzt werden, das aus rekombinanten Quellen stammt, das sich vom natürlichen, menschlichen Afamin beispielsweise durch seine Glykosylierungsweise, seinen Lipidgehalt oder aber gar in der Aminosäuresequenz unterscheiden kann.

Neben üblichen pharmazeutischen Trägerstoffen können dem erfindungsgemäßen Präparat auch weitere pharmazeutisch wirksame Komponenten zugesetzt werden, z.B. solche, die für eine bestimmte Indikation bereits bekannt sind.

Die Erfindung wird anhand der nachfolgenden Beispiele und der Zeichnungsfiguren, auf die sie selbstverständlich nicht eingeschränkt sein soll, näher erläutert. Es zeigen:
Fig. 1 die Glutaminsynthetase-Aktivität nach 5-tägiger Behandlung mit 3-NP in Gegenwart von verschiedenen Schutzstoffen;
Fig. 2a Afamin und die Kombination von Afamin mit Vitamin E, die vor neuronalem Zelltod schützt, ausgelöst durch partiellen Serumentzug auf 2 %;
Fig. 2b Afamin und die Kombination von Afamin mit Vitamin E, die vor neuronalem Zelltod schützt, ausgelöst durch 100 µM H₂O₂;
Fig. 2c Afamin und die Kombination von Afamin mit Vitamin E, die vor neuronalem Zelltod schützt, ausgelöst durch 20 µM präaggregiertes beta-Amyloid Peptid;
Fig. 3 eine Scatchard-Plot-Analyse der Bindung von Vitamin E an Afamin.

### Beispiele

### Beispiel 1: 3NP (3-Nitroproprionsäure)-induzierte Reduktion von Glutaminsynthetase (GS)-Aktivität

Wegen der mechanistischen und pathologischen Ähnlichkeiten zwischen 3-NP-Läsionen und der Huntingtonschen Erkrankung (Huntington's Disease; HD) wurde 3-NP als alternatives HD-Modell vorgeschlagen. Malonat und 3-NP sind Inhibitoren der Succinatdehydrogenase, welche einen Energieverlust und Läsionen erzeugen, welche ähnlich denen in HD beobachteten sind. Systemische Verabreichung von 3-NP verursacht eine progressive lokomotorische Verschlechterung, die der von HD entspricht. 3-NP verursacht sehr selektive striatale Degenerierung. Es unterscheidet sich mechanistisch von excitotoxischen Läsionen, indem 3-NP den mitochondrialen Citratzyklus irreversibel inhibiert und zu verringerten ATP-Werten und erhöhten Lactatkonzentrationen führt.

### Assay:

Nach Extraktion von Gehirnspheroiden und einer einwöchigen Kultivierung wurde D,L-alpha-Tocopherol (0,1 mM) dem Medium zugefügt. Zwei Wochen alte Spheroiden wurden in einer Platte mit sechs Vertiefungen ausplattiert. Kurz nach der Exponierung wurde das Medium durch 2 ml frisches Medium ersetzt. Die Spheroiden wurden an 3-NP (0,5 und 5 mM, pH auf 7 bis 8 eingestellt) oder die Protektoren Vitamin E (0,1 mM), Glutathion (1 mM), DTT (0,25 mM), L-NAME (N(G)-Nitro-L-Alaninmethylester) (0,1 mM), Afamin (ohne Vitamin E) (0,3 und 3 µg/ml) oder Kombinationen von 3-NP mit den Protektoren während 5 Tage ausgesetzt. Nach 2 Tagen wurden frisch hergestelltes 3-NP und Protektoren zum Medium zugefügt (Mathews et al., J.Neurosci, 18(1) (1998), 156-163); Borlongan et al., Neurosci Biohav. Res. 21(3) (1997), 289-293).

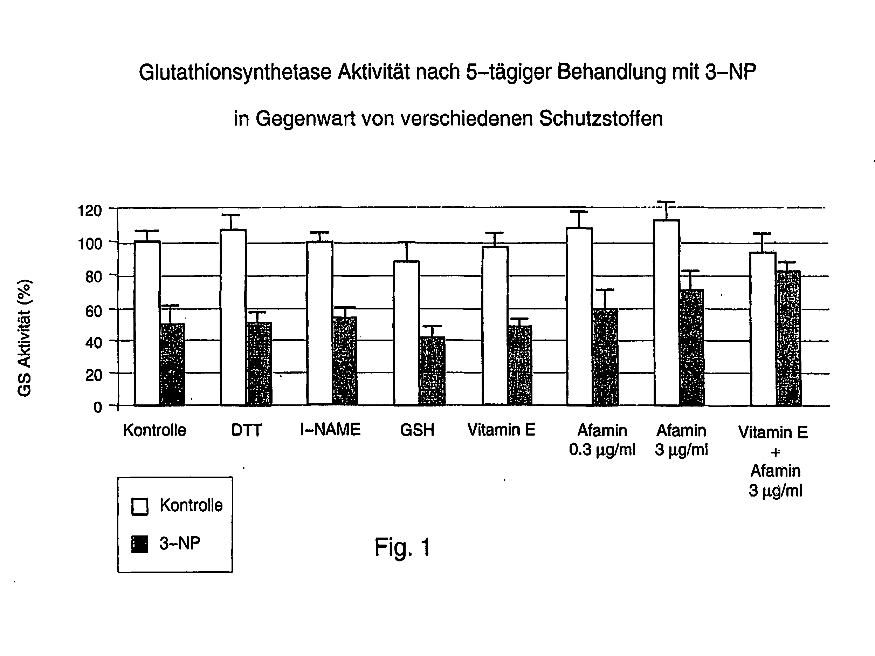

Die Ergebnisse sind in Fig. 1 dargestellt. Hier zeigt sich, dass von den getesteten Substanzen Afamin den weitaus größten Effekt beim Schutz der Glutaminsynthetase (GS, ein astrozytisches Enzym, das sehr sensitiv gegenüber oxidativen Stress ist) gegen Inaktivierung durch 3NP hat. Wie erwähnt stellt dieses System mit 3NP ein anerkanntes Modell für Excitotoxizität dar und dient als Modell für die Huntingtonsche Erkrankung.

### Beispiel 2: Neuroprotektiver Effekt in isolierten cortikalen Neuronen von Hühnerembryonen

Der neuroprotektive Effekt von Afamin wurde durch Untersuchungen an isolierten cortikalen Neuronen von Hühnerembryonen untersucht. Dabei wurde die Wirkung von Afamin in einem Low-Serum-Assay und in zwei Läsionen-Assays, nämlich in einem β-Amyloid-, und einem H₂O₂-Läsionen-Assay, getestet.

Nach dem Low Serum-Assay oder Läsionen-Assay wird der MTT-Assay zur Überprüfung der Lebensfähigkeit von neuronalen Zellen herangezogen (MTT = 3-(4,5-Dimethylthiazol-2-yl)-2,5, diphenyltriazoliumbromid). Dies ist ein geeignetes Modell, welches äußerst reproduzierbar ist. Der Test erlaubt auch die Untersuchung einer hohen Anzahl an Proben und Dosis-abhängige Unterschiede in der Wirkung einer Substanz können evaluiert werden.

Die neuronale Lebensfähigkeit von Zellen in vitro kann mittels dieses colometrischen MTT-Test festgestellt werden. Der MTT-Test (oder MTT-Assay) basiert auf der metabolischen Reduktion von MTT, einer Substanz mit gelber Farbe, zu dunkelblauen Formazankristallen durch mitochondriale Dehydrogenasen (z.B. Succinatdehydrogenase). Die Kristalle werden aufgelöst und die spektrometrische Absorption wird bei geeigneter Wellenlänge gemessen. Es konnte gezeigt werden, dass tote Zellen nicht dazu in der Lage sind MTT zu spalten; ruhende Zellen erzeugen weniger Formazan, daher kann dieser Assay für die Quantifizierung der Lebensfähigkeit von Zellen herangezogen werden, da diese Reaktion nur durch lebende aktive Zellen katalysiert wird (Mosmann et al., J.Immunol.Meth. 65(1983), 55; Bernabei et al., Hemat.Oncol.7 (1989), 243; Barltrop et al., Bioorg & Med.Chem.Lett.1 (1991), 611; Cory et al., Cancer Commun. 3 (1991), 207).

Im Detail wurden hiefür isolierte cortikale Neuronen von 8 (Läsion-Assays) oder 9 (Low Serum Assay) Tage alten Hühnerembryonen (White Leghorn oder Lohman Brown-Hybrid-Stamm) verwendet.

Die Neuronen wurden präpariert, indem Ethanol-behandelte Eier aufgebrochen und die Embryonen in eine Plastikschale gegeben wurden. Nach Dekapitierung wurden die Hemisphären entfernt und gesammelt. Loses Gewebe und meningiale Membranen wurden entfernt und die Hemisphären mechanisch dissoziiert.

80 µl Zellsuspension, enthaltend 6 x 10⁵ Zellen/ml Nährmedium, wurden zu jeder Vertiefung der Mikroplatte, die bereits 80 µl Medium mit oder ohne den Substanzen enthielt, gegeben. Die Platten wurden bei 37°C, 95% Feuchtigkeit und 5% CO₂ ohne Medienwechsel gehalten (bis 8 DIV).

Das "Low Serum"-Medium enthält 100 ml DEMEM mit 1 g Glucose/l, 2% fötales Kälberserum, 0,01% Gentamycin und 2 mM L-Glutamin. Das Nährmedium für die "Läsionen-Assays" enthält 100 ml DMEM mit 4,5 g Glucose/l, 5% fötales Kälberserum, 0,01% Gentamycin und 2 mM L-Glutamin.

Bei DIV 8 wurden die Zellen mit 20 µM während 72 Stunden präaggregiertem β-Amyloid-Peptid (Aβ₂₅₋₃₅; Sigma) oder 100 µM H₂O₂ für 24 h behandelt.

Nach dem Ende jedes Experiments wurde die Lebensfähigkeit der Kulturen mit Hilfe von MTT-Assay mit einem Plattenleser bei 570 nm getestet.

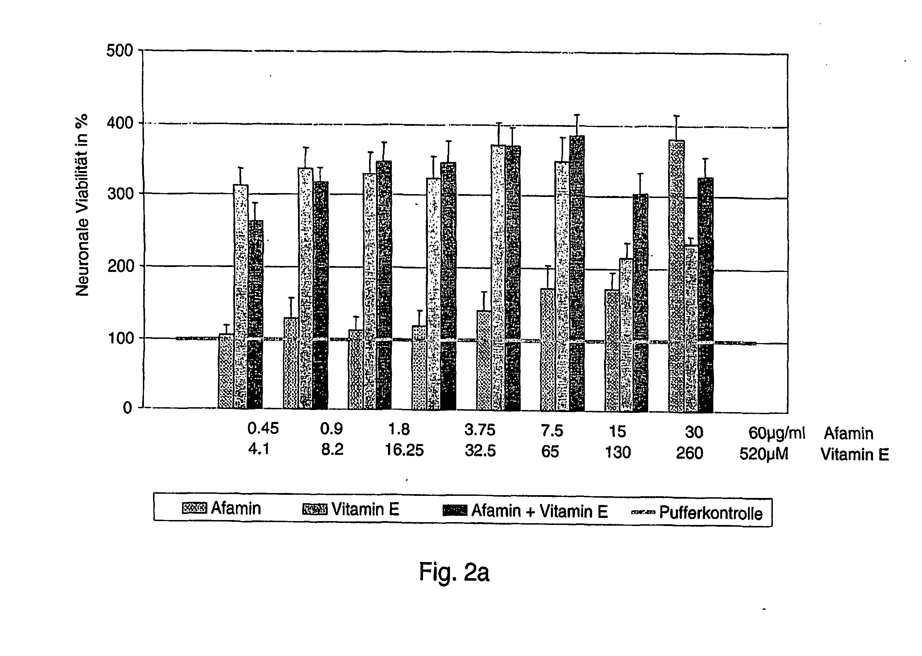

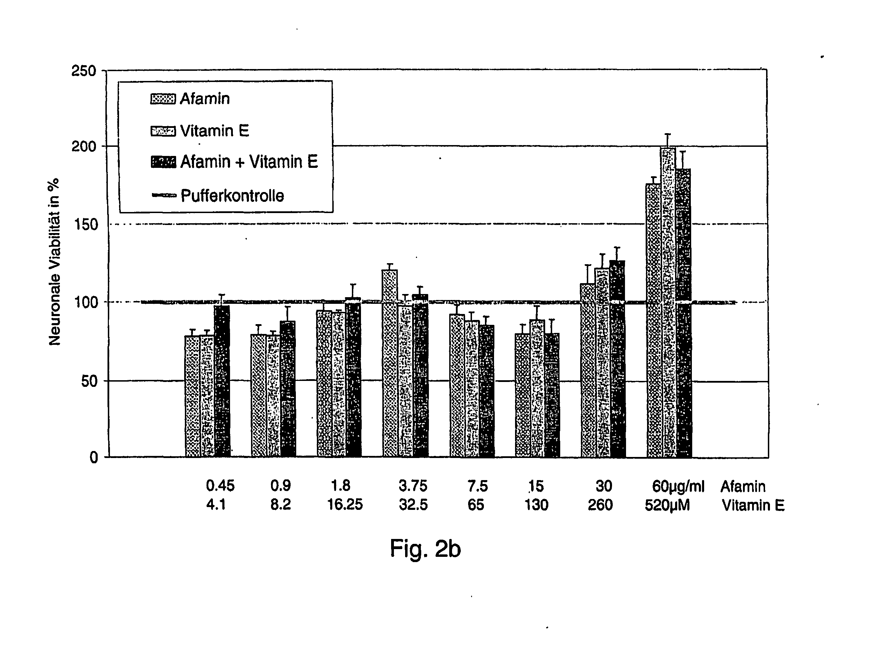

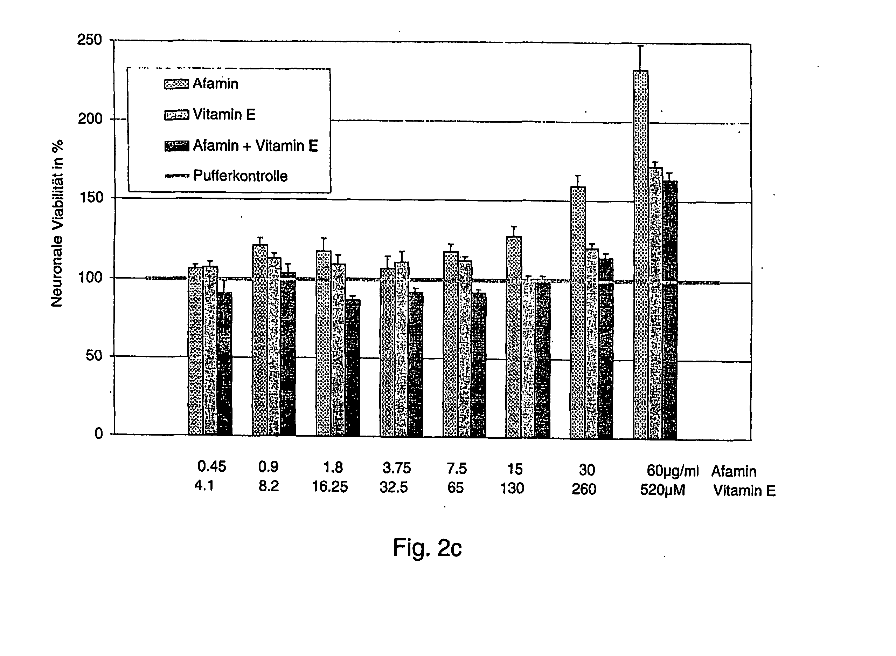

Die Ergebnisse sind in Fig. 2a-c dargestellt und zeigen, dass Afamin, Vitamin E und vor allem die Kombination dieser beiden Substanzen die Apoptose in 2% "low serum"-Medium (Fig. 2a), beziehungsweise die Schädigung durch β-Amyloid-Peptid oder H₂O₂, welche bei den Kontrollversuchen beobachtet werden konnte, reduziert werden konnte.

## Patentansprüche

1. Verwendung von Afamin, insbesondere in Kombination mit Vitamin E, zur Herstellung einer präparation zur Behandlung von neurodegenerativen Erkrankungen.

2. Verwendung nach Anspruch 1, **dadurch gekennzeichnet, dass** eine Präparation zur Behandlung der Alzheimerschen Erkrankung hergestellt wird.

3. Verwendung nach Anspruch 1 , **dadurch gekennzeichnet, dass** eine Präparation zur Behandlung der Buntington-Erkrankung hergestellt wird.

4. Verwendung nach Anspruch 1 , **dadurch gekennzeichnet, dass** eine Präparation zur Behandlung der Amyotropischen Lateralsklerose hergestellt wird.

5. Verwendung nach Anspruch 1 , **dadurch gekennzeichnet, dass** eine Präparation zur Behandlung von neurodegenerativen Demenzen hergestellt wird.

6. Verwendung nach Anspruch 1 , **dadurch gekennzeichnet, dass** eine Präparation zur Behandlung akuter neurodegenerativer Erkrankungen, insbesondere zur Behandlung von Epilepsie, Trauma und zerebraler Ischämie, hergestellt wird.

7. Verwendung nach Ansprach 1, **dadurch gekennzeichnet, dass** eine Präparation zur Behandlung der Parkinsonschen Erkrankung hergestellt wird.

8. Verwendung nach Anspruch 1, **dadurch gekennzeichnet, dass** eine Präparation zur Behandlung der der Alzheimerschen Erkrankung ähnlichen Veränderungen bei Patienten mit Down-Syndrom hergestellt wird.

9. Verwendung nach Anspruch 1, **dadurch gekennzeichnet, dass** eine Präparation zur Behandlung von Nebenwirkungen einer mit oxidativem Stress verbundenen Applikation von Medikamenten hergestellt wird.

10. Verwendung nach Anspruch 1, **dadurch gekennzeichnet, dass** eine neuroprotektive Präparation hergestellt wird.

11. Vitamin-E-Präparation mit erhöhter antioxidativer Wirkung, **dadurch gekennzeichnet, dass** sie Afamin enthält.

12. , Vitamin-E-Präparation nach Anspruch 11, **dadurch gekennzeichnet, dass** sie rekombinantes Afamin enthält.

13. Vitamin-E-Präparation nach Anspruch 11 oder 12, **dadurch gekennzeichnet, dass** sie rekombinantes Afamin mit derivatisierten Seitenketten oder hinzugefügten Peptidketten enthält, oder Teile von Afamin oder derivatisiertem Afamin enthält.

14. Vitamin-E-Präparation nach einem der Ansprüche 11 bis 13, **dadurch gekennzeichnet, dass** sie einen pharmazeutisch akzeptablen Trägerstoff enthält.

## Claims

1. The use of Afamin, in particular in combination with vitamin E, for the production of a preparation for treating neurodegenerative diseases.

2. The use according to claim 1, **characterized in that** a preparation for treating Alzheimer's Disease is produced.

3. The use according to claim 1, **characterized in that** a preparation for treating Huntington's Disease is produced.

4. The use according to claim 1, **characterized in that** a preparation for treating amyotrophic lateral sclerosis is produced.

5. The use according to claim 1, **characterized in that** a preparation for treating neurodegenerative dementias is produced.

6. The use according to claim 1, **characterized in that** a preparation for treating acute neurodegenerative diseases, in particular for treating epilepsy, trauma and cerebral ischaemia, is produced.

7. The use according to claim 1, **characterized in that** a preparation for treating Parkinson's Disease is produced.

8. The use according to claim 1, **characterized in that** a preparation is produced for treating changes occurring in patients afflicted with Down syndrome, which changes are similar to Alzheimer's Disease.

9. The use according to claim 1, **characterized in that** a preparation for treating side-effects of an application of medicaments associated with oxidative stress is produced.

10. The use according to claim 1, **characterized in that** a neuroprotective preparation is produced.

11. A vitamin E preparation with an increased antioxidant activity, **characterized in that** it contains Afamin.

12. A vitamin E preparation according to claim 11, **characterized in that** it contains recombinant Afamin.

13. A vitamin E preparation according to claim 11 or 12, **characterized in that** it contains recombinant Afamin with derivatized side chains or added peptide chains, or parts of Afamin or parts of derivatized Afamin.

14. A vitamin E preparation according to any one of claims 11 to 13, **characterized in that** it contains a pharmaceutically acceptable carrier substance.

## Revendications

1. Utilisation d'afamine, en particulier en combinaison avec la vitamine E, pour la production d'une préparation destinée au traitement de maladies neurodégénératives.

2. Utilisation suivant la revendication 1, **caractérisée en ce qu'**une préparation destinée au traitement de la maladie d'Alzheimer est produite.

3. Utilisation suivant la revendication 1, **caractérisée en ce qu'**une préparation destinée au traitement de la maladie de Huntington est produite.

4. Utilisation suivant la revendication 1, **caractérisée en ce qu'**une préparation destinée au traitement de la sclérose latérale amyotrophique est produite.

5. Utilisation suivant la revendication 1, **caractérisée en ce qu'**une préparation destinée au traitement de démences neurodégénératives est produite.

6. Utilisation suivant la revendication 1, **caractérisée en ce qu'**une préparation destinée au traitement de maladies neurodégénératives aiguës, en particulier, en particulier au traitement de l'épilepsie, du traumatisme et de l'ischémie cérébrale, est produite.

7. Utilisation suivant la revendication 1, **caractérisée en ce qu'**une préparation destinée au traitement de la maladie de Parkinson est produite.

8. Utilisation suivant la revendication 1, **caractérisée en ce qu'**une préparation destinée au traitement de modifications analogues à la maladie d'Alzheimer chez des patients présentant le syndrome de Down est produite.

9. Utilisation suivant la revendication 1, **caractérisée en ce qu'**une préparation destinée au traitement d'effets secondaires d'une administration de médicaments liée à un stress oxydatif est produite.

10. Utilisation suivant la revendication 1, **caractérisée en ce qu'**une préparation neuroprotectrice est produite.

11. Préparation de vitamine E à action antioxydative élevée, **caractérisée en ce qu'**elle contient de l'afamine.

12. Préparation de vitamine E suivant la revendication 11, **caractérisée en ce qu'**elle contient de l'afamine recombinée.

13. Préparation de vitamine E suivant la revendication 11 ou 12, **caractérisée en ce qu'**elle contient de l'afamine recombinée à chaînes latérales dérivatisées ou à chaînes peptidiques ajoutées, ou des parties d'afamine ou d'afamine dérivatisée.

14. Préparation de vitamine E suivant l'une des revendications 11 à 13, **caractérisée en ce qu'**elle contient un support acceptable du point de vue pharmaceutique.
